**Europäisches Patentamt**

**European Patent Office**

**Office européen des brevets**

(11) Veröffentlichungsnummer: **0 320 767 B1**

## EUROPÄISCHE PATENTSCHRIFT

(45) Veröffentlichungstag der Patentschrift: **10.03.93**

(51) Int. Cl.⁵: **A61B 3/02**

(21) Anmeldenummer: **88120418.4**

(22) Anmeldetag: **07.12.88**

Die Akte enthält technische Angaben, die nach dem Eingang der Anmeldung eingereicht wurden und die nicht in dieser Patentschrift enthalten sind.

(54) **Verfahren zur Gesichtsfeldprüfung.**

(30) Priorität: **18.12.87 DE 3742945**

(43) Veröffentlichungstag der Anmeldung:
**21.06.89 Patentblatt 89/25**

(45) Bekanntmachung des Hinweises auf die
Patenterteilung:
**10.03.93 Patentblatt 93/10**

(84) Benannte Vertragsstaaten:
**AT BE CH FR GB IT LI LU NL SE**

(56) Entgegenhaltungen:
**DE-C- 3 135 383**
**US-A- 3 883 234**
**US-A- 4 334 738**

**"Halbautomatische Methode zur subjektiven Messung des binokularen Akkomodation" ; J. HESSEN, H. KRUEGER in Biomed. Technik, Band 24 (Ergänzungsband), S. 327/328.**

(73) Patentinhaber: **Weber, Jörg, Dr.-Med.**
**Auf dem Mühlenacker 40**
**W-5030 Hürth-Hermülheim(DE)**

(72) Erfinder: **Weber, Jörg, Dr.-Med.**
**Auf dem Mühlenacker 40**
**W-5030 Hürth-Hermülheim(DE)**

(74) Vertreter: **Goddar, Heinz J., Dr. et al**
**FORRESTER & BOEHMERT Franz-Joseph-Strasse 38**
**W-8000 München 40 (DE)**

**Beschreibung**

Die Erfindung betrifft ein Verfahren zur Gesichtsfeldprüfung mit den Merkmalen des Oberbegriffs von Patentanspruch 1.

Aus der DE-PS 31 35 383 ist ein derartiges Verfahren bzw. eine Vorrichtung bekannt, die jedoch den Nachteil hat, daß der Schwellenwert, also die Intensität, bei der ein Lichtpunkt im Gesichtsfeld gerade noch gesehen wird, nicht bestimmt wird.

Es sind weiter Verfahren bzw. Vorrichtungen zur Gesichtsfeldprüfung bekannt, bei denen die Intensität des Lichtpunktes stufenweise verändert werden kann. Bei den bekannten Vorrichtungen ist der Zeitaufwand zur Ermittlung des Schwellenwerts unter Umständen jedoch erheblich, da bei der erforderlichen feinen Abstufung der Intensitäten eine sehr große Vielzahl von Ansteuerung der Lampe erfolgen muß, wobei die zu untersuchende Person frühzeitig ermüdet, ohne daß die Genauigkeit wesentlich verbessert wird.

Ein Beispiel für ein Verfahren, das mit einer abnehmenden Stufung arbeitet, ist in dem Aufsatz "Halbautomatische Methode zur subjektiven Messung der binokularen Akkomodation" von J. Hessen und H. Krueger in Biomedizinische Technik, Band 24 (Ergänzungsband, Juni 1979, Seiten 327/328 zum Messen der Akkomodationsfähigkeit des Auges beschrieben.

Der Erfindung liegt die Aufgabe zugrunde, das Verfahren der eingangs genannten Gattung derart weiterzubilden, daß eine genaue Bestimmung des Schwellenwertes in relativ geringer Zeit möglich wird.

Diese Aufgabe wird von einem Verfahren nach Patentanspruch 1 gelöst. Vorteilhafte Ausgestaltungen sind Gegenstand der abhängigen Ansprüche.

Die vorgeschlagene Ausgestaltung des Verfahrens hat den Vorteil, daß die Zeitdauer einer Gesichtsfelduntersuchung ohne Minderung der Genauigkeit insbesondere bei schlechten Werten erheblich reduziert wird.

Im folgenden wird die Arbeitsweise der vorgeschlagenen Vorrichtung anhand von Beispielen erläutert.

Zur Verdeutlichung sollen zunächst die Begriffe "Lichtintensität" und "Schwellenwert" definiert werden. Als Lichtintensität (I) (Leuchtdichte) wird die Helligkeit des Lichtpunkts gemessen in Candela pro Quadratzentimeter ($cd/m^2$) angegeben. Als Schwellenwert (S) (auch als Empfindlichkeitswert bezeichnet) wird der dekadische Logarithmus des Kehrwertes der Intensität I (bezogen auf eine gerätespezifische, frei festzulegende Maximalintensität $I_0$) multipliziert mit dem Faktor 10 bezeichnet. Es ergibt sich somit

$$S = 10 * -lg(I/I_0)$$

nach I aufgelöst

$$I = I_0 * 10^{-S/10}.$$

Eine Erhöhung der Intensität bedeutet somit eine Erniedrigung der abgefragten Empfindlichkeit (Schwelle). 0 dB ist somit die höchste prüfbare Intensität, nämlich $I_0$ und entspricht damit der niedrigsten prüfbaren Schwelle.

Bei den bekannten Steuervorrichtungen ist die Differenz aufeinanderfolgender Schwellenwertstufen gleich, was wegen des logarithmischen Charakters der Schwellenwerte ein konstantes Verhältnis aufeinanderfolgender Intensitäten bedeutet.

Anders als bei den bekannten Vorrichtungen wählt die Steuereinrichtung bei der Vorrichtung nach der Erfindung die Intensitätsfaktoren, denen Schwellenwert-Differenzen entsprechen, wobei die Abstufung in einer Phase mit zunehmender Intensität zunimmt und in einer Phase mit abnehmender Intensität abnimmt. Lediglich beispielhaft sei eine entsprechende Tabelle dargestellt:

0 6 11 15 19 22 25 28 30 32 33 34 35 dB.

Die Abstufungen nehmen somit von 6 dB auf 1 dB kontinuierlich ab (natürlich sind auch Tabellen mit Zwischenstufen von 0,5 dB-Werten möglich, um gleichmäßigere Übergänge zu schaffen). Oben angegebene Tabelle dient lediglich zur Erläuterung des Grundprinzips.

Bei der ersten Phase soll - auch dies lediglich beispielhaft - die Abstufung aufeinanderfolgender Lichtpunkte 2 Stufen obiger Tabelle betragen, in der zweiten Phase beträgt die Abstufung 1 Stufe. Eine Abfolge könnte sich damit wie folgt ergeben:

25(+), 30(+); 33(-) // 32(-), 30(+) // Ergebnis: 31 dB

oder aber

25(-), 19(-), 11(-), 0(+) // 6(+), 11(-) // Ergebnis: 8,5 dB.

Bei dem weiteren Vorschlag, die Steuereinrichtung derart einzurichten, daß in jeder Phase unabhängig von einem etwaigen Antwortwechsel wenigstens zwei Lichtpunkte in abgestufter Intensität aufleuchten läßt, wird eine höhere Sicherheit erreicht, da eine Falschantwort das Ergebnis weniger oder gar nicht verfälscht. Unter der Annahme, daß bei dem zweiten der beiden oben angegebenen Beispiele der eine Schwellenwert von 19 dB unrichtig mit "gesehen" beantwortet wird (+ !) ergibt sich folgender Ablauf ohne die Weiterbildung:

25(-), 19(+) // 22(-) // Ergebnis: 20,5 dB

und mit der Weiterentwicklung

25(-), 19(+) // 22(-), 19(-), 15(-), 11(-), 6(+) // Ergebnis: 8,5 dB.

Der Vorschlag nach der Erfindung, die Steuereinrichtung so auszubilden, daß die Faktoren aufeinanderfolgender Intensitäten derart ungleich sind, so daß bei zunehmender Intensität der Faktor der Intensitätsabstufung innerhalb einer Phase zunimmt und mit abnehmender Intensität abnimmt, ermöglicht es, mit einer deutlich geringeren Anzahl von Lichtpunktdarbietungen den Schwellenwert mit nur geringfügig uneingeschränkter Genauigkeit zu bestimmen. Damit wird das Verhältnis der Zahl der Darbietungen des Lichtpunkts im Verhältnis zu der erzielten Genauigkeit erheblich verbessert.

Nach einem bevorzugten Ausführungsbeispiel der Erfindung ist es möglich, zur Zeitersparnis nach jeder Phase die Untersuchung abzubrechen und den sich bei Abschluß der letzten vollendeten Phase ergebenden Schwellenwert zu bestimmen. Bei einem derartigen vorzeitigen Abbruch ist die Genauigkeit der Bestimmung zwar geringer, dies kann jedoch bei vielen Fällen in Kauf genommen werden.

**Patentansprüche**

1. Verfahren zur Gesichtsfeldprüfung, bei dem über das Gesichtsfeld der zu untersuchenden Person verteilte Lichtpunkte aufleuchten, wobei die Lichtpunkte steuerbar in logarithmisch abgestuften Intensitätsfaktoren aufleuchten, denen Schwellenwert-Differenzen entsprechen, wobei die zu untersuchende Person die Antwort "gesehen" oder "nicht gesehen" durch Auslösen eines Signals gibt und bei einer Antwort "gesehen" der nächstfolgende Lichtpunkt an der gleichen Stelle mit geringerer Intensität aufleuchtet und bei einer Antwort "nicht gesehen" der nächstfolgende Lichtpunkt an der gleichen Stelle mit höherer Intensität aufleuchtet und wobei bei einem Antwortwechsel in eine nächste Phase mit zunehmender bzw. abnehmender Intensität des Lichtpunkts gewechselt wird, in der die Intensitätsabstufung aufeinanderfolgender Lichtpunkte geringer als in der vorangehenden Phase ist, dadurch gekennzeichnet, daß aufeinanderfolgende Lichtpunkte innerhalb einer Phase mit zunehmender Intensität mit zunehmender Intensitätsabstufung und in einer Phase mit abnehmender Intensität mit abnehmender Intensitätsabstufung aufleuchten.

2. Verfahren nach Anspruch 1, dadurch gekennzeichnet, daß in jeder Phase unabhängig von einem etwaigen Antwortwechsel wenigstens zwei Lichtpunkte in abgestufter Intensität aufleuchten.

3. Verfahren nach Anspruch 1 oder Anspruch 2, dadurch gekennzeichnet, daß es nach jeder Phase beendbar ist.

**Claims**

1. A method of testing the field of vision, in which light spots distributed over the field of vision of the subject light up, the light spots lighting up controllably in logarithmically graduated intensity factors, having corresponding threshold value differences, the subject giving the answer "seen" or "not seen" by triggering a signal and, in the case of the answer "seen", the next light spot lighting up at the same place with reduced intensity and in the event of the answer "not seen" the next light spot lighting up at the same place with higher intensity, and in the event of a change of answer there is a change to a next phase with increasing or decreasing intensity of the light spot respectively, in which next phase the intensity graduation of successive light spots is less than in the preceding phase, characterized in that successive light spots within a phase of increasing intensity light up with decreasing intensity graduation while in a phase with decreasing intensity they light up with decreasing intensity graduation.

2. A method according to claim 1, characterised in that at least two light spots light up in graduated intensity in each phase independently of any change of answer.

3. A method according to claim 1 or 2, characterized in that it can be terminated after each phase.

**Revendications**

1. Procédé de périmétrie dans lequel s'allument des points lumineux répartis dans le champ visuel de la personne examinée, ces points lumineux s'allumant de manière commandable à des facteurs d'intensité échelonnés logarithmiquement auxquels correspondent des différences de seuils, la personne examinée donnant la réponse "vu" ou la réponse "pas vu" en déclenchant un signal, et, en cas de réponse "vu", le point lumineux suivant s'allumant au même endroit avec une plus faible intensité et en cas de réponse "pas vu", le point lumineux suivant s'allumant au même endroit avec une plus forte intensité, et, en cas de changement de réponse, ayant lieu le passage à une phase suivante à intensité croissante ou décroissante du point lumineux dans laquelle l'échelonnement des intensités des points lumineux successifs est plus petit que dans la phase précédente, caractérisé par le fait que les points lumineux successifs, dans une phase à intensité croissante, s'allument avec échelonnement des intensités croissant et, dans une phase à intensité décroissante, s'allument avec échelonnement des intensités décroissant.

2. Procédé selon la revendication 1, caractérisé par le fait que dans chaque phase, indépendamment d'un éventuel changement de réponse, au moins deux points lumineux s'allument à intensité échelonnée.

3. Procédé selon l'une des revendications 1 et 2, caractérisé par le fait qu'il peut y être mis fin après chaque phase.